# EUROPEAN PATENT SPECIFICATION

(11) **EP 1 227 812 B1**
(45) Date of publication and mention of the grant of the patent: **18.04.2007**
(21) Application number: 00968725.2
(22) Date of filing: 05.10.2000
(51) Int. Cl.: A61K 31/451, A61P 29/00, A61P 27/00

(54) **TOPICAL NASAL TREATMENT USING DESLORATADINE AND MOMETASONE FUROATE**
TOPISCHE NASENBEHANDLUNG MIT DESLORATADIN UND MOMETASON FUROAT
TRAITEMENT NASAL TOPIQUE AVEC LA DESLORATADINE ET FUROATE DU MOMETASONE

(30) Priority: 08.10.1999 US 415083
(43) Date of publication of application: 07.08.2002
(73) Proprietor: SCHERING CORPORATION, Kenilworth, NJ 07033-0530 (US)
(72) Inventor: ANDRZEJEWSKI, Stephen, J., Mendham, NJ 07945 (US); KREUTNER, William, West Paterson, NJ 07424 (US)
(74) Representative: Adams, Harvey Vaughan John
(86) International application number: PCT/US2000/027463
(87) International publication number: WO 2001/026658

(56) References cited:
- WO-A-00/51605
- US-A- 5 939 426
- REICHMUTH, DANIEL ET AL: "Present and potential therapy for allergic rhinitis A review" BIODRUGS (2000), 14(6), 371-387 , XP001001196
- SEGURA T. ET AL: "Allergic rhinitis: Basic pathophysiology and therapeutic strategies." CANADIAN JOURNAL OF ALLERGY AND CLINICAL IMMUNOLOGY, (1999) 4/7 (318-330) , XP000998728
- CARDELUS IGNASI ET AL: "Anticholinergic effects of desloratadine, the major metabolite of loratadine, in rabbit and guinea-pig iris smooth muscle." EUROPEAN JOURNAL OF PHARMACOLOGY, vol. 374, no. 2, 18 June 1999 (1999-06-18), pages 249-254, XP001001230 ISSN: 0014-2999
- LIPPERT, U. ET AL: "Pharmacological modulation of IL-6 and IL-8 secretion by the H1-antagonis decarboethoxy-loratadine and dexamethasone by human mast and basophilic cell lines" EXP. DERMATOL. (1995), 4(4, PT. 2), 272-6 , XP001001161

## Description

### FIELD OF THE INVENTION

The present invention relates to the treatment of nasal disorders, and more particularly to topical treatments using an antihistamine, or together with a corticosteroid.

### INTRODUCTION TO THE INVENTION

Allergic disorders are very prevalent throughout the world, causing a very significant cost to society for therapy and in the form of worker absenteeism and decreased worker productivity. These Illnesses are frequently manifested in the form of acute or chronic rhinitis, sometimes also described, respectively, as seasonal and perennial rhinitis. The symptoms of allergic rhinitis may include any number of: reddening of the eyes; ocular secretions; nasal congestion; ocular and palatial irritation; sneezing; and hypersecretion. Symptoms typically occur very promptly following exposure to allergens, of which the most common are grass, pollens and mold spores. The incidence of allergic rhinitis is greater during the spring and summer months, but some persons suffer the symptoms during the entire year, with exacerbations during the spring and summer.

When airborne allergens interact with specific antibodies attached to mast cell membranes, the mast cells react by releasing histamine, in a process called degranulation. Histamine is a potent vasodilator and increases microvascular permeability, allowing plasma flow into extravascular spaces and thereby causing edema.

Antihistamines, administered systemically alone or together with sympathomimetic decongestants, have traditionally been the drugs of choice in treating allergic rhinitis. Other forms of therapy have included the use of topically applied cromolyn sodium, hypertonic salt solutions or immunotherapy.

Antihistamines have also been applied directly to nasal tissues. Reports of experiments using the antihistamine azatadine are given by R. M. Naclerio et al., "in Vivo Model for the Evaluation of Topical Antiallergic Medications." *Archives of Otolaryngology,* Vol. 110, pp. 25-27, 1984, and by A. G. Togias et al., "Demonstration of Inhibition of Mediator Release from Human Mast Cells by Azatadine Base, In Vivo and In Vitro Evaluation," *Journal of the American Medical Association,* Vol. 255, pp.225-229, 1986. A currently available commercial product provides azelastine in an aqueous based nasal spray form.

In addition, Hagen et al, in U.S. Patent 4,787,612 discloses nasal corticosteroid therapy as an effective means of treating allergic rhinoconjunctivitis, and is herein incorporated by reference in its entirety. European Patent Application 0 780 127 A1 discloses nasal spray compositions containing both a glucocorticosteroid and an antihistamine possessing leukotriene inhibiting properties, for treating rhinoconjunctivitis. International Patent Application WO 97/46243 discloses pharmaceutical compositions for nasal administration, comprising a glucocorticoid and a fast acting antihistamine, for treating allergic rhinitis.

Notwithstanding the many disclosures in the area of allergic rhinitis, there remains a need for compositions which can be applied topically to provide improved symptomatic rhinitis relief with a very low incidence of adverse effects, particularly sedation.

### SUMMARY OF THE INVENTION

The present invention provides the use of desloratadine in the manufacture of a medicament for treating rhinitis by a combination therapy comprising topical application of desloratadine and mometasone furoate. In a second aspect, the invention provides the use of mometasone furoate in the manufacture of a medicament for treating rhinitis by a combination therapy comprising topical application of desloratadine and mometasone furoate.

The active substances may be administered together or separately. However, it is preferred that they be administered together, most preferably in a single formulation.

Treatment can be effected using intranasal compositions which are aqueous or nonaqueous. Thus, in accordance with a further aspect of the invention there is provided a fluid composition characterised by containing destoratadine and mometasone furoate, in a pharmaceutically acceptable carrier,

While it is possible that some portion of the topically applied drugs will develop systemic concentrations, such as by absorption through nasal or other mucous membranes or through the gastrointestinal tract after being swallowed, the desired treatment effect at least initially results from an external contact of the applied drugs with the nasal tissues.

### DETAILED DESCRIPTION OF THE INVENTION

The symptoms of allergic rhinitis usually include ocular and palatial irritation, ocular secretions, reddening of the eyes, sneezing, mucoid hypersecretion and itching. A later manifestation of the condition is nasal congestion. The allergic rhinitis can be of the seasonal or perennial types. Other types of rhinitis, such as vasomotor rhinitis, exhibit some of the same symptoms and typically respond in varying degrees to similar treatment.

Various "antihistamines" have been known since the 1940's. These agents, while differing in their chemical structures, are histamine H₁ receptor antagonists which bind to those receptors and thereby prevent the binding of histamine. The more modem antihistamines have a greatly reduced potential for crossing the blood-brain barrier, so do not possess the well known sedation potential of many of the older agents.

Among the known antihistamines are three having a close chemical structure relationship: azatadine, loratadine and desloratadine (also known as "descarboethoxyloratadine" or "DCL"). Their common structure is as follows: 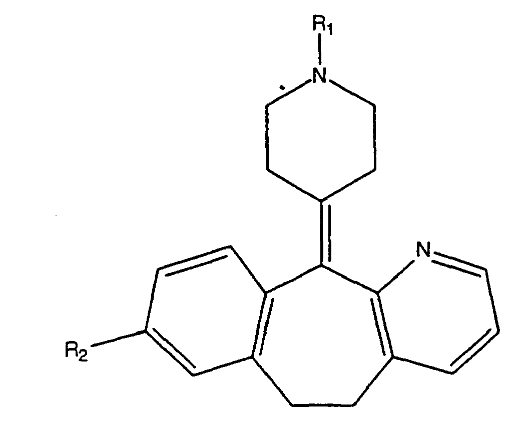 where azatadine has a -CH₃ group for R₁ and hydrogen for R₂ ; loratadine has a -C(O)OCH₂CH₃ group for R₁ and chlorine for R₂ ; and desloratadine has hydrogen for R₁ and chlorine for R₂.

Azatadine is a potent topical (i.e., locally acting) antihistamine, as recognized in the above-described papers by Naclerio et al. and Togias et al. However, the drug has a marked sedation potential in many people; the Togias et al. paper reports drowsiness in one of the eight subjects tested. Both loratadine and desloratadine have a greatly reduced sedation potential, as compared to azatadine, so would generally be preferred. Loratadine is asserted in European Patent Application 0 780 127 A1 as being useful in topical compositions, but the present inventors have not found a useful degree of topical activity for this drug. The literature does not report anything concerning topical activity for desloratadine, although International Patent Application WO 96/20708 does mention topical dosing; this topical dosing, given the context, apparently relates to an alternative means for achieving effective systemic amounts of the drug. Therefore, it is considered that topical (i.e., local) antihistaminic efficacy is not a predictable property of compounds having this general chemical structure.

Corticosteroids which are useful in the present invention have potent glucocorticoid activity and weak mineralocorticoid activity. These agents affect the inflammatory condition that eventually results from the allergic response. The corticosteroid agent presently preferred in the invention is mometasone the mention of any base drug specifically including hydrates and other addition compounds thereof, as well as pharmaceutically acceptable salts thereof. The following representative drug compounds are currently commercially available in the United States: betamethasone acetate, dipropionate and sodium phosphate; dexamethasone base, acetate and sodium phosphate; beclomethasone dipropionate; flunisolide base; triamcinolone acetonide, diacetate and hexacetonide; fluticasone propionate; mometasone foroate and furoate monohydrate; and budesonide base. Due to its very low systemic bioavailability, mometasone (generally used in the form of its furoate, or as the furoate monohydrate) is particularly advantageous.

Mometasone furoate monohydrate is commercially formulated as an aqueous nasal spray suspension, and sold using the NASONEX^{™} name.

Mometasone furoate and desloratadine will each be administered in a pharmaceutically acceptable intranasal carrier. Preferably, the active substances would be present in an aqueous carrier, dissolved therein to provide an easily attainable physical stability during storage and dose reproducibility. However, certain corticosteroids are quite insoluble in aqueous media. Desloratadine also has limited solubility in aqueous media. If preparation of a solution is not feasible, then the aqueous carrier will be used to form a suspension formulation having one or more active ingredients uniformly dispersed therein, in the form of a micronized powder. Either type of aqueous formulation can be conveniently packaged in spray bottles, preferably those having metering pump delivery means; it is highly desirable to use such devices to deliver reproducible amounts of the formulation, since the drugs are quite potent.

The desired degree of isotonicity of the aqueous compositions of this invention may be accomplished using, for example, sodium chloride or other pharmaceutically acceptable agents such as dextrose, boric acid, citric acid, sodium tartrate, sodium phosphate, potassium phosphate, propylene glycol or other inorganic or organic solutes or mixtures thereof. Sodium chloride is preferred particularly for buffers containing sodium ions. Further examples of sodium chloride equivalents are disclosed in A. R. Gennaro, Ed., *Remington's Pharmaceutical Sciences, 18th Ed.,* Mack Publishing Co., Easton, Pennsylvania U.S.A., (1990) at pages 1491-1497.

Viscosity of the compositions may be maintained at the selected level using a thickening agent. Suitable thickening agents include, for example, xanthan gum, carrageenan, blends of microcrystalline cellulose and sodium carboxymethyl cellulose, and the like, including pharmaceutically acceptable salts thereof. Mixtures of thickening agents may also be used. The concentration of the thickener will vary, depending upon the agent selected. The important consideration is to use an amount that will achieve the desired viscosity. Viscous compositions are normally prepared from solutions or suspensions by the addition of such thickening agents. For suspension products, agents which produce thixotropic compositions are preferable, since such compositions promote suspension stability; many particulates tend to aggregate when they do not remain suspended, and become difficult to redisperse.

Preferred compositions within the scope of this invention will contain from about 0.01 % to about 10% of a humectant to inhibit drying of the mucous membranes and to prevent irritation. A variety of pharmaceutically-acceptable humectants are useful including, for example, sorbitol, propylene glycol, glycerol or mixtures thereof. As with the thickeners, the concentration will vary with the selected agent, although the presence or absence of these agents, or their concentration, is not an essential feature of the invention.

A pharmaceutically-acceptable preservative is generally employed to increase the shelf life of the compositions of the present invention. Benzyl alcohol is suitable, although a variety of other preservatives including, for example, parabens, phenylethyl alcohol, thimerosal, chlorobutanol, phenylmecuric acetate and benzalkonium chloride may be employed. A suitable concentration of the preservative will be from 0.001 % to 2% by weight, although there may be appreciable variation depending upon the agent selected. Mixtures of preservatives may also be used.

A variety of additional, optional ingredients may be added to the topical nasal compositions of the present Invention. These additional ingredients include various polymers for aiding the film-forming properties and substantivity of the formulation, antioxidants, and agents suitable for aesthetic purposes such as fragrances.

Another preferred form for nasally administered drugs is the pressurized metered dose inhaler. These products usually utilize a low-boiling chlorofluorocarbon or fluorohydrocarbon propellant, into which the drug substances may be dissolved (sometimes with the aid of a cosolvent, such as an alcohol) or suspended. Since chlorofluorocarbon propellants are regarded as being harmful to the environment, it is presently preferred to use a fluorohydrocarbon alternative such as 1,1,1,2-Tetrafluoroethane ("HFC-134a") or 1,1,1,2,3,3,3-Heptafluoropropane ("HFC-227"). The formulation is contained in a pressure-resistant container, such as a polymeric, glass or aluminum canister, and fitted with a metering valve which releases a fixed volume of the composition with each actuation. Of course, additives are commonly used to provide valve lubrication, assist in maintaining a stable solution or suspension, and for other functions. For these formulations, it is frequently desirable to chemically modify the drug substance; beclomethasone dipropionate, for example, is typically used in pressurized aerosols in the form of a micronized addition compound (such as a solvate or clathrate) with a molecule of the chlorofluorocarbon or fluorohydrocarbon propellant.

Surfactants are frequently incorporated into pressurized aerosol formulations to aid in maintaining a stable dispersion of components and to assist with maintaining proper metering valve function. The commonly used oleic acid is suitable, in concentrations which will provide up to about 50 µg with each actuation of the valve. Since it is always desired to minimize the amounts of inactive substances in a medication, the lowest concentration which yields the desired effect should be used. Other useful surfactants include, without limitation thereto, sorbitan trioleate, cetyl pyridinium chloride, soya lecithin, polyoxyethylene (20) sorbitan monolaurate, polyoxyethylene (10) stearyl ether, polyoxyethylene (2) oleyl ether, polyoxyethylene-polyoxypropylene-ethylenediamine block copolymers, polyoxyethylene (20) sorbitan monostearate, polyoxyethylene-polyoxypropylene block copolymers, castor oil ethoxylate and mixtures of any two or more. In choosing a surfactant system, it is generally preferable that the surfactant is soluble in the propellant or in an alcohol-propellant solution. For any desired surfactant, simple experiments to measure drug delivery reproducibility can be employed to identify the optimum amount of surfactant for any given formulation and delivery system.

The choice of suitable carrier forms will depend on the exact nature of the particular nasal dosage form required, e.g., whether the drug(s) will be formulated into a nasal solution (for use as drops or as a spray), a nasal suspension, a nasal ointment, a nasal gel or another nasal form. Some of these forms, however, will be less susceptible to reproducible dose measurement and uniform application to the nasal mucosa. In making the choices of dosage form, it should be kept in mind that the object of treatment is to affect allergic and inflammatory processes in nasal tissues, and not to provide any maximized systemic levels of the drugs. It will be advantageous to use a composition which will distribute over a maximum area of the nasal mucosal tissues, thus generally favoring the sprayed dosage forms.

In the present invention, Mometasone furoate is typically present in a topical nasal dosage form at concentrations about 0.001 % to about 0.2%, preferably about 0.01 % to about 0.1 %. Desloratadine is typically present at concentrations about 0.05% to about 10%, preferably about 0.2% to about 5%.

Preferably the composition is applied to the nasal mucosa via topical application of a safe and effective amount to treat the undesired nasal symptoms. The amount of the desloratadine and Mometasone furoate and frequency of topical application to the nasal mucosa may vary, depending upon personal or medical needs, but it is suggested, as an example, that the application ranges from about once per day to about four times daily, preferably no more than twice daily, and most preferably once daily. Typically dosage units will be prepared to deliver from about 10 µg to about 1000 µg of Mometasone furoate and from about 100 µg to about 5 mg of desloratadine per spray actuation or metered dose "puff." A typical dose comprises one to four sprays per nostril. For convenience considerations, it is generally preferred that each dose of the two drugs is administered at the same point in time, more preferably with both present in a single formulation. However, Mometasone furoate may require dosing at different intervals than does desloratadine, so different formulations and dosing schedules may be used.

Optional ingredients which are useful in the present invention include topically active decongestants. Decongestants useful in the present invention include the sympathomimetic amines, some examples of which are ephedrine, levmetamfetamine, propylhexedrine, xylometazoline, phenylephrine, naphazoline, oxymetazoline and pharmaceutically acceptable salts thereof. Mixtures of such decongestants are also useful.

When used in the compositions of the present invention, the sympathomimetic agents may be incorporated at concentrations about 0.01 % to about 0.5%, preferably from about 0.05% to about 0.1%.

The compositions of the present invention may optionally also contain an antiallergic. Suitable antiallergics include, but are not limited to, cromolyn, which is believed to inhibit mast cell degranulation.

Similarly, mucolytics such as acetylcysteine and antimuscarinics such as ipratropium bromide and tiotropium bromide may be incorporated into the compositions of the present invention.

Also of optional use in the compositions of the present invention are nonopiate analgesics such as oxaprozin, acetaminophen, acetylsalicylic acid, ibuprofen, etedolac, fenbuprofen, fenoprofen, flurbiprofen, indomethacin, ketoprofen, naproxen, pharmaceutically acceptable salts thereof, optically active racemates thereof and mixtures thereof.

Synthetic opiate analgesics such as oxycodone, buprenorphine, meperidine, methadone, propoxyphene, pentazocine, pharmaceutically acceptable salts thereof and mixtures thereof may be beneficially incorporated.

Various aromatic components (e.g., aldehydes and esters) may also be used. These aromatics include, for example, menthol, camphor, eucalyptol, benzaldehyde (cherry, almond), citral (lemon, lime), neral, decanal (orange, lemon), aldehyde C-B, aldehyde 0-9 and aldehyde 0-12 (citrus fruits); tolyl aldehyde (cherry almond); 2,6-dimethyl-octanal (green fruit); and 2-dodecenal (citrus, mandarin). Mixtures of such aromatics can also be used. It also frequently is desired to incorporate any of the well-known natural or synthetic flavoring agents, including, without limitation, mint or fruit flavors.

In the following examples, Examples 1 and 2 describe and demonstrate embodiments within the scope of the present invention. Examples 3 and 4 are not within the scope of the invention and are provided by way of illustrating analogous formulations to there of the invention.

### EXAMPLE 1

A 12 Kg batch of an aqueous nasal spray is prepared, using the following ingredients:

| Component | Grams |
|---|---|
| Mometasone furoate monohydrate, micronized | 6 |
| Desloratadine, micronized | 1 |
| Avicel^{™} RC-591 | 240 |
| Glycerin | 252 |
| Citric acid | 24 |
| Sodium citrate | 33.6 |
| Polysorbate 80 | 1.2 |
| Benzalkonium chloride | 2.4 |
| Phenylethyl alcohol | 30 |

The composition is prepared using the following procedure: (a) the Avicel RC-591 (a mixture of microcrystalline cellulose and cellulose gum, sold by FMC Corporation, Philadelphia, Pennsylvania U.S.A.) is dispersed in about 6 Kg of water and the glycerin is added; (b) the citric acid and sodium citrate is dissolved in about 250 grams of water, then added to the dispersion of step a; (c) the polysorbate 80 is dissolved in about 400 grams of water and, with continuous stirring, both of the drug substances are added ; (d) the step c mixture is added to the step b dispersion; and (e) the benzalkonium chloride and phenylethyl alcohol are dispersed in a small amount of water and added to the step d mixture, then sufficient water is added to provide a total batch weight of 12 Kg.

This suspension may be administered using a typical pump spray device, preferably delivering a volume of 100 microliters per actuation.

### EXAMPLE 2

Pressurized metered dose inhaler formulations are prepared using the ingredients mometasone furoate ("MF"), desloratadine ("DCL"), oleic acid ("OLEIC"), ethanol ("ETOH"), and the propellant 1,1,1,2,3,3,3-Heptafluoropropane ("HFC-227"), where the numerical representations given in the following table are weight percentages.

| FORMULA | MF | DCL | OLEIC | ETOH | HFC-227 |
|---|---|---|---|---|---|
| A | 0.112 | 0.5 | 0.001 | 2.497 | 96.889 |
| B | 0.028 | 0.5 | 0 | 1.75 | 96.889 |
| C | 0.112 | 1 | 0.011 | 2.497 | 96.379 |
| D | 0.448 | 0.25 | 0 | 0 | 99.302 |
| E | 0.112 | 0.1 | 0 | 2.497 | 97.290 |
| F | 0.448 | 0.1 | 0.011 | 1.5 | 93.054 |
| G | 0.224 | 0.05 | 0.011 | 4 | 93.22 |
| H | 0.028 | 0.1 | 0.001 | 3 | 94.371 |
| I | 0.028 | 0.02 | 0.011 | 2.499 | 97.442 |

The aerosol formulations are prepared using any of the following procedures, or any equivalent thereof:
(1) all components except the HFC-227 are charged into aerosol canisters, the canisters are chilled to a temperature below the boiling point of the propellant, the required amount of liquid propellant is added and metering valves are crimped onto the canisters;
(2) all components except the HFC-227 are charged to aerosol canisters, metering valves are crimped onto the canisters and liquid propellant is charged into the canisters through the valves; or
(3) a quantity of the formulation is prepared by mixing required amounts of the components in a suitable chilled environment, below the propellant boiling point, and the well-mixed formulation is charged into aerosol canisters which have previously been fitted with metering valves.

### EXAMPLE 3

Pressurized aerosol metered dose inhalers are prepared using the following formulations, where "BDP" is beclomethasone dipropionate, "DCL" is desloratadine, "ETOH" is ethanol and "HFC-134a" is the propellant 1,1,1,2-Tetrafluoroethane.

| FORMULA | BDP | DCL | ETOH | HFC-134a |
|---|---|---|---|---|
| 1 | 0.1 | 2 | 5 | 92.9 |
| 2 | 0.3 | 0.01 | 8 | 91.69 |

The drug substances are mixed with the ethanol and stirred or subjected to ultrasonic agitation to dissolve at least the BDP, and the mixture is placed into aerosol canisters. After chilling the canisters to a temperature below the boiling point of the propellant, the liquid propellant is measured into the canisters. Metering valves, designed to deliver 50 µL per actuation, are then crimped onto the canisters.

### EXAMPLE 4

The topical efficacy of desloratadine for preventing histamine-induced vascular permeability increases in nasal mucosa is compared with that for loratadine.

Guinea pigs are anesthetized and Evans blue dye is administered intravenously, in amounts of 30 mg per Kg of body weight. After 5 minutes, a 100 µL quantity of either a vehicle (1 % by volume dimethylsulfoxide in an aqueous buffer which is 10 mM Na₂HPO₄/NaH₂PO₄ dissolved in 0.9% wt. NaCl, adjusted to pH 7.2) or a solution of antihistamine in the vehicle is instilled into the nares, using 50 µL per nostril. The nasal airway is occluded with a tracheal cannula, inserted in the cephalic direction, and ten minutes following the instilling of antihistamine or vehicle a perfusion of 1 mM histamine in the above-described buffer, at a rate of 0.2 mL/minute, is run through the cannula for 30 minutes and the perfusate fluid exiting the external nares is collected. The concentration of dye in the perfusate is analyzed spectrophotometrically, higher dye concentrations indicating that higher degrees of microvascular permeability are caused by the exogenous histamine exposure.

Results are as in the following table, showing mean values of percent inhibition of vasopermeability, as compared to that measured with only vehicle, for up to eight animals per dose tested.

| Drug | Dose, µg | % Inhibition |
|---|---|---|
| Loratadine | 1 | 13 |
| | 3 | 41 |
| | 10 | 49 |
| | 30 | 10 |
| | | |
| Desloratadine | 0.1 | 0 |
| | 0.3 | 36 |
| | 1 | 50 |
| | 3 | 69 |
| | 10 | 56 |

Desloratadine is approximately an order of magnitude more active in this test, than is loratadine. The seemingly anomalous results for the highest amounts of loratadine may be due to those amounts of the compounds not being completely soluble in 100 µL of the vehicle.

## Claims

1. Use of desloratadine in the manufacture of a medicament for treating rhinitis by a combination therapy comprising topical application of desloratadine and mometasone furoate.

2. Use of mometasone furoate in the manufacture of a medicament for treating rhinitis by a combination therapy comprising topical application of desloratadine and mometasone furoate.

3. The use of either of Claim 1 or Claim 2, **characterised by** the desloratadine and mometasone furoate being present in separate dosage forms.

4. The use of either of Claim 1 or Claim 2, **characterised by** the desloratadine and mometasone furoate being present in a single dosage form.

5. The use of any of Claims 1 to 4, **characterised by** the medicament being in the form of an aqueous nasal spray,

6. The use of any of Claims 1 to 4, **characterised by** the medicament being in the form of a pressurised metered dose inhaler.

7. A fluid composition **characterised by** containing desloratadine and mometasone furoate, in a pharmaceutically acceptable carrier.

8. The composition of Claim 7, **characterised by** one or both of desloratadine and mometasone furoate being present as suspended particles.

9. The composition of either of Claim 7 or Claim 8, **characterised by** the carrier comprising an aqueous medium.

10. The composition of either of Claim 7 or Claim 8, **characterised by** the carrier comprising a low-boiling chlorofluorocarbon or fluorohydrocarbon propellant.

## Patentansprüche

1. Verwendung von Desloratadin zur Herstellung eines Medikaments zur Behandlung von Rhinitis durch eine Kombinationstherapie, die die topische Anwendung von Desloratadin und Mometasonfuroat beinhaltet.

2. Verwendung von Mometasonfuroat zur Herstellung eines Medikaments zur Behandlung von Rhinitis durch eine Kombinationstherapie, die die topische Anwendung von Desloratadin und Mometasonfuroat beinhaltet.

3. Verwendung nach Anspruch 1 oder Anspruch 2, **dadurch gekennzeichnet, dass** das Desloratadin und Mometasonfuroat in separaten Dosierungsformen vorliegen.

4. Verwendung nach Anspruch 1 oder Anspruch 2, **dadurch gekennzeichnet, dass** das Desloratadin und Mometasonfuroat in einer einzelnen Dosierungsform vorliegen.

5. Verwendung nach einem der Ansprüche 1 bis 4, **dadurch gekennzeichnet, dass** das Medikament in Form eines wässrigen Nasensprays vorliegt.

6. Verwendung nach einem der Ansprüche 1 bis 4, **dadurch gekennzeichnet, dass** das Medikament in Form eines mit Druck beaufschlagten Dosierinhaliergeräts vorliegt.

7. Fluidzusammensetzung, **dadurch gekennzeichnet, dass** sie Desloratadin und Mometasonfuroat in einem pharmazeutisch annehmbaren Träger enthält.

8. Zusammensetzung nach Anspruch 7, **dadurch gekennzeichnet, dass** einer oder beide von Desloratadin und Mometasonfuroat als suspendierte Teilchen vorliegen.

9. Zusammensetzung nach Anspruch 7 oder Anspruch 8, **dadurch gekennzeichnet, dass** der Träger ein wässriges Medium enthält.

10. Zusammensetzung nach Anspruch 7 oder Anspruch 8, **dadurch gekennzeichnet, dass** der Träger ein niedrig siedendes Chlorfluorkohlenstoff- oder Fluorkohlenwasserstoff-Treibmittel enthält.

## Revendications

1. Emploi de desloratadine dans la fabrication d'un médicament conçu pour le traitement d'une rhinite par thérapie combinée comprenant l'administration topique de desloratadine et de furoate de mométasone.

2. Emploi de furoate de mométasone dans la fabrication d'un médicament conçu pour le traitement d'une rhinite par thérapie combinée comprenant l'administration topique de desloratadine et de furoate de mométasone.

3. Emploi conforme à l'une ou l'autre des revendications 1 et 2, **caractérisé en ce que** la desloratadine et le furoate de mométasone se présentent en formes posologiques distinctes.

4. Emploi conforme à l'une ou l'autre des revendications 1 et 2, **caractérisé en ce que** la desloratadine et le furoate de mométasone se présentent en une forme posologique unique.

5. Emploi conforme à l'une des revendications 1 à 4, **caractérisé en ce que** le médicament se présente sous forme de spray nasal aqueux.

6. Emploi conforme à l'une des revendications 1 à 4, **caractérisé en ce que** le médicament se présente en inhalateur-doseur pressurisé.

7. Composition fluide, **caractérisée en ce qu'**elle contient, dans un véhicule pharmacologiquement admissible, de la desloratadine et du furoate de mométasone.

8. Composition conforme à la revendication 7, **caractérisée en ce que** l'un des deux composants, desloratadine et furoate de mométasone, ou les deux s'y trouvent à l'état de particules en suspension.

9. Composition conforme à l'une ou l'autre des revendications 7 et 8, **caractérisée en ce que** le véhicule comprend un milieu aqueux.

10. Composition conforme à l'une ou l'autre des revendications 7 et 8, **caractérisée en ce que** le véhicule comprend un agent propulseur de type chlorofluorocarbure ou fluorohydrocarbure, à bas point d'ébullition.
